(19) Europäisches Patentamt
European Patent Office
Office européen des brevets

(11) Veröffentlichungsnummer: **0 248 393 B1**

(12) # EUROPÄISCHE PATENTSCHRIFT

(45) Veröffentlichungstag der Patentschrift: **07.08.91**

(51) Int. Cl.5: **C07D 307/48**, C07D 307/58, C07D 333/22, C07D 333/32, C07D 207/33, C07D 207/36

(21) Anmeldenummer: **87107940.6**

(22) Anmeldetag: **02.06.87**

Die Akte enthält technische Angaben, die nach dem Eingang der Anmeldung eingereicht wurden und die nicht in dieser Patentschrift enthalten sind.

(54) **Verfahren zur Herstellung von acylierten Heteroaromaten an zeolithischen Katalysatoren.**

(30) Priorität: **05.06.86 DE 3618964**

(43) Veröffentlichungstag der Anmeldung: **09.12.87 Patentblatt 87/50**

(45) Bekanntmachung des Hinweises auf die Patenterteilung: **07.08.91 Patentblatt 91/32**

(84) Benannte Vertragsstaaten: **BE DE FR GB NL**

(56) Entgegenhaltungen:
**DE-A- 2 616 583**
**US-A- 2 458 521**

**CHEMICAL ABSTRACTS, Band 101, Nr. 15, 08 Okt. 1984, Columbus, Ohio, USA ANISIMOV, A V; BABAITSEV, V S; VIKTOROVA, E A : "Oxyethylation of heteroaromatic thiols", Seite 695, Spalte 2, Z'fassung 130547y.**

(73) Patentinhaber: **BASF Aktiengesellschaft**
**Carl-Bosch-Strasse 38**
**W-6700 Ludwigshafen(DE)**

(72) Erfinder: **Lermer, Helmut, Dr.**
**D 3,4**
**W-6800 Mannheim 1(DE)**
Erfinder: **Hoelderich, Wolfgang, Dr.**
**Mannheimer Strasse 18 c**
**W-6710 Frankenthal(DE)**
Erfinder: **Schwarzmann, Matthias, Dr.**
**Carl-Bosch-Strasse 54**
**W-6703 Limburgerhof(DE)**

EP 0 248 393 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 69, Nr. 25, 16 Dez. 1968, Columbus, Ohio, USA SAPOZHNIKOVA, E A; SULTANOV, A S : "Recyclodehydration of tetrahydrofurfuryl alcohol on aluminosilicate", Seite 9962, Spalte 2, Z'fassung 106416e

CHEMICAL ABSTRACTS, Band 71, Nr. 23, 08 Dez. 1969, Columbus, Ohio, US DANILOVA, T A; KHALIL, F; TITS-SKVORTSOVA, I N ; RYBNIKOVA, A A ; TRIPPLER, S ; SHTROBEL, M; AST, E; BAKH, G; OSIPENKO, TS D ; "Conversions of thiophene compounds in the presence of an aluminosilicate catalyst",Seite 296, Spalte 2, Z'fassung 112186n.

CHEMICAL ABSTRACTS, Band 75, Nr. 25, 20 Dez 1971, Columbus, Ohio, US V I CHERNOV; A V MASHKINA; "Activity of amorphous and crystalline aluminosilicates during transformation of alkylthiophenes", S 307, Spalte 2, Z'fassung 151616e .

G A OLAH "Friedel-Crafts and Related Reactions", Band III, Teil 1, 1964 INTERSCIENCE PUBLISHERS, New York-London-Sydney, Seiten 88,89.

## Beschreibung

Die vorliegende Anmeldung betrifft ein Verfahren zur Herstellung von acylierten fünfgliedrigen Heteroaromaten durch Umsetzung von entsprechenden Heteroaromaten mit gängigen Acylierungsmitteln an zeolithischen Katalysatoren.

Die häufigste Anwendung von Zeolithen in der Acylierung von Aromaten stellt die Anthrachinon-Synthese aus Benzol und Phthalsäureanhydrid dar.

Aus DE-PS 26 33 458 ist bekannt, daß man bei Einsatz von teilweise substituierten Phthalsäureanhydriden entsprechende Anthrachinone ohne Zugabe von Benzol erhält. Hierbei verwendet man als Katalysatoren mit Mg, Ca, Ba oder Zn modifizierte Zeolithe.

Aus DE-PS 26 16 583 ist ein Verfahren zur Acylierung nichthydroxilierter Aromaten bekannt. Als Katalysator werden z.B. Y-Zeolithe verwendet, die mit Lanthaniden dotiert sind. Anwendungen sind die Herstellung von 2,4-Dimethylacetophenon, Benzophenon oder Anthrachinon.

Aus (US-PS 2 458 521) ist die Acylierung von Thiophen in einem kontinuierlichen Gasphasenverfahren mit einem Katalysator, bestehend aus $SiO_2$ und wasserunlöslichem Metalloxid, z.B. $ZrO_2$ oder $ThO_2$, natürlichem Ton und synthetischen Zusammensetzungen von $SiO_2$ und MeO (Montmorillonit) bekannt. Mit diesen Katalysatoren werden niedrige Ausbeuten, z.B. 23 % maximal (Beispiel 1) erzielt, wobei man jeweils das 2-Acetylthiophen erhält.

Auch die Acylierung von Phenolen an Mordeniten,

$$\gamma\text{-}Al_2O_3$$

und weiteren kristallinen und amorphen Aluminosilicaten bei Temperaturen von 380-450 °C ist bekannt.

2-Acetylthiophen läßt sich z.B. aus Thiophen und Acetylchlorid mit $SnCl_4$ in Benzol darstellen (Beilstein 17, II, 314).

2-Acetylfuran kann unter Verwendung von HI, $H_3PO_4$, $BF_3$, $SnCl_4$ oder $ZnCl_2$ als Katalysator synthetisiert werden (Beilstein 17, III, 4500).

2-Acetylpyrrol entsteht bei Einsatz von $HClO_4$ oder $POCl_3$ aus Pyrrol und Acetanhydrid (Beilstein 21, III, 3437).

Doch verursacht die homogene Friedel-Crafts-Acylierung die bekannten Nachteile, wie hohe Kosten hinsichtlich des Katalysators, der Abtrennung des Katalysators und der Entsorgung. Dazu kommt die Problematik der Toxizität des Katalysators wie $BF_3$, der Übergangskomplexe und des zu entsorgenden Katalysators.

Es wurde nun gefunden, daß man acylierte fünfgliedrige Heteroaromaten der Formel Ia oder Ib

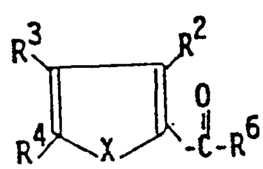 (Ia), 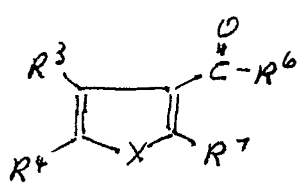 (Ib),

worin

X = O oder $NR^5$,

$R^1$-$R^4$ = H-, Alkyl- mit 1 bis 12 Kohlenstoffatomen, Alkenyl- mit bis 12 Kohlenstoffatomen, Alkoxyl- mit 1 bis 12 Kohlenstoffatomen, Aryl-, Aralkyl- oder Alkylarylreste,

$R^5$ = H-, Alkyl mit 1 bis 12 Kohlenstoffatomen, Alkenyl- mit bis 12 Kohlenstoffatomen, Aryl-, Aralkyl- oder Alkylarylreste und

$R^6$ = H-, Alkyl- mit 1 bis 12 Kohlenstoffatomen, Alkenyl- mit bis 12 Kohlenstoffatomen, Aryl-, Aralkyl-, Alkylaryl- oder Carboxyreste bedeuten,

aus fünfgliedrigen Heteroaromaten der Formel II

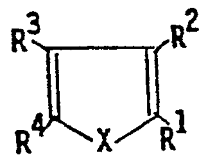 (II),

in der
X = O oder $NR^5$
$R^1$-$R^4$ = H-, Alkyl- mit 1 bis 12-Kohlenstoffatomen, Alkenyl- mit bis 12 Kohlenstoffatomen, Alkoxyl- bis 1 bis 12 Kohlenstoffatomen, Aryl-, Aralkyl- oder Alkylarylreste
$R^5$ = H-, Alkyl mit 1 bis 12 Kohlenstoffatomen, Alkenyl- mit bis 12 Kohlenstoffatomen, Aryl-, Aralkyl- oder Alkylarylreste bedeuten,
wobei mindestens einer der Reste $R^1$ oder $R^2$ Wasserstoff ist,
mit gängigen Acylierungsmitteln der Formel III

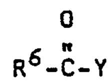 (III),

in der
$R^6$ die obengenannte Bedeutung hat und
Y = Halogenid-, Alkoxy-, Acyloxy-, Hydroxy-, Amid-, oder Carboxyreste bedeuten,
in Gegenwart von zeolithischen Katalysatoren herstellt.

Die Umsetzung von Furan, Thiophen oder Pyrrol mit Essigsäureanhydrid zu Acetylfuran bzw. - thiophen bzw. -pyrrol aus einem Zeolith-Katalysator läßt sich beispielsweise durch folgende Formelgleichung wiedergeben:

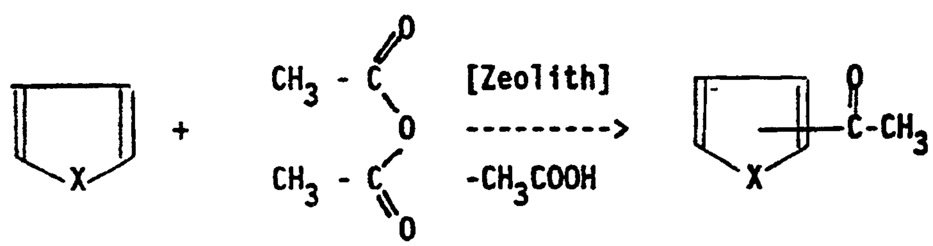


X = O, S, NH.

Die Nachteile der bekannten Verfahren werden durch das erfindungsgemäße Verfahren vermieden.

Die für das erfindungsgemäße Verfahren eingesetzten fünfgliedrigen Heteroaromaten sind Furane, Thiophene und Pyrrole der oben angegebenen Formel II, z.B. Furan, Thiophen, Pyrrol, 2- oder 3-Methylfuran, 2- oder 3-Methylthiophen-, 1- oder 2- oder 3-Methylpyrrol, 2,3-Dimethylfuran, 2,3-Dimethylpyrrol, 1,3-Dimethylpyrrol, 1,2,3-Trimethylpyrrol, 2-Methoxyfuran, 2-Methoxythiophen, 2-Ethylfuran, 2-Ethylthiophen, 2-Methoxypyrrol, 1-Methyl-2-methoxypyrrol, 1-Ethyl-2-methylpyrrol, 1-Ethyl-2,3-dimethylpyrrol, 2-Isopropylfuran, 2-Isopropylthiophen, 2-Isopropylpyrrol, 2-Butylfuran, 2-Butylthiophen, 2-Butylpyrrol, 1,2,3,4-Tetramethylpyrrol, 2,3,4-Trimethylfuran, 2,3,4-Trimethylthiophen, Cyclohexylfuran, Cyclohexylthiophen, Cyclohexylpyrrol, Cyclopentylfuran, Cyclopentylthiophen, Cyclopentylpyrrol, Cyclohexenylfuran, Cyclohexenylthiophen, Cyclohexenylpyrrol, Vinylfuran, Vinylthiophen, Vinylpyrrol, 2-Methyl-3-vinylfuran, 2-Methyl-3-vinylthiophen, Isopropenylfuran, Isopropenylthiophen, 1-Methyl-3-isopropenylpyrrol, Benzylfuran, Benzylthiophen, Benzylpyrrol, Phenylfuran, Phenylthiophen oder Phenylpyrrol.

Die für das erfindungsgemäße Verfahren eingesetzten gängigen Acylierungsmittel sind z.B. Essigsäurechlorid, Essigsäure, Essigsäureanhydrid, Essigsäuremethylester, Essigsäureethylester, Essigsäureamid, Propionsäurechlorid, Propionsäure, Propionsäureanhydrid, Buttersäurechlorid, Buttersäure, Buttersäureanhydrid, Isobuttersäure, Isobuttersäurechlorid, Isobuttersäureanhydrid, Crotonsäure, Crotonsäureanhydrid, Isoprensäure, Valeriansäure, Valeriansäurechlorid, Capronsäure, Capronsäureester, Pivalinsäure, Pivalinsäurechlorid, Acrylsäure, Acrylsäuremethylester, Methacrylsäure, Methacrylsäureester, Penten-3-säuremethylester, Penten-2-säuremethylester,Penten-4-säuremethylester, Sorbinsäure, Sorbinsäuremethylester, Benzoesäure, Benzosäurechlorid, Benzoesäuremethylester, o-, m-, p-Fluorbenzoesäure, o-, m-, p-Fluorbenzoesäurechlorid, o-, m-, p-Chlorbenzoesäure, o-, m-, p-Methylbenzoesäure-, o-, m-, p-Methylbenzoesäurechlorid, o-, m-, p-Isopropylbenzoesäure, o-, m-, p-Isopropylbenzoesäurechlorid, o-, m-, p-Methoxybenzoesäure, o-, m-, p-Methoxybenzoesäurechlorid, 2,3-Dimethyl-, 2,3-Difluor-, 2,3-Dichlor-, 2,3-Dimethoxybenzoesäure bzw. -benzoesäurechlorid, Phenylessigsäure, Phenylessigsäurechlorid, Phenylessigsäureanhydrid, Zimtsäure, Zimtsäurechlorid, Malonsäure, Malonsäureanhydrid, Phthalsäure, Phthalsäureanhydrid, Terephthalsäure und Terephthalsäurechlorid.

Als Katalysatoren für das erfindungsgemäße Verfahren werden zeolithische Katalysatoren eingesetzt. Zeolithe sind kristalline Aluminosilikate, die eine hochgeordnete Struktur mit einem starren dreidimensionalen Netzwerk von $SiO_4$- oder $AlO_4$-Tetraedern besitzen, die durch gemeinsame Sauerstoffatome verbunden sind. Das Verhältnis der Si- und Al-Atome zu Sauerstoff beträgt 1:2. Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekeln besetzt.

In den Zeolithen können anstelle von Aluminium auch andere Elemente wie B, Ga, Fe, Cr, V, As, Sb oder Be in das Gitter eingebaut werden, oder das Silicium kann durch ein vierwertiges Element wie Ge, Ti, Zr, Hf ersetzt werden.

Entsprechend ihrer Struktur werden die Zeolithe in verschiedene Gruppen unterteilt. So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder Y.

Für das erfindungsgemäße Verfahren in Betracht kommende Katalysatoren sind Zeolithe aus der Mordenit-Gruppe oder Engporige Zeolithe vom Erionit- bzw. Chabasit-Typ oder Zeolithe vom Faujasit-Typ, z.B. Y-, X-oder L-Zeolithe. In diese Gruppe von Zeolithen gehören auch die sogenannten "ultrastabilen" Zeolithe des Faujasit-Typs, d.h. dealumierte Zeolithe. Verfahren zur Herstellung solcher Zeolithe sind beschrieben in "Catalysis by Zeolites" Band 5 aus Studies in Surface Science and Catalysis, ed. B. Imelik et al., Elsevier Scientific Publishing Comp. 1980, S. 203 und "Crystal Structures of Ultra-stable Faujasites" Advances in Chemistry Series Nr. 101, American Chemical Society Washington, DC, S 226 ff (1971) und in US-PS 4 512 961.

Besonders vorteilhaft verwendet man Zeolithe vom Pentasiltyp. Diese haben als Grundbaustein einen aus $SiO_4$-Tetraedern aufgebauten Fünfring gemeinsam. Sie sind durch ein hohes $SiO_2/Al_2O_3$-Verhältnis gekennzeichnet, sowie durch Porengrößen, die zwischen denen der Zeolithe vom Typ A und denen vom Typ X oder Y liegen.

Die verwendbaren Zeolithe können unterschiedliche chemische Zusammensetzung aufweisen, geeignet sind z.B. Alumino-, Boro-, Eisen-, Beryllium-, Gallium-, Chrom-, Arsen-, Antimon- und Wismutsilikatzeolithe oder deren Gemische sowie Alumino-, Boro-, Gallium-, Eisengermanat-zeolithe oder deren Gemische. Insbesondere eignen sich die Alumino-, Boro- oder Eisensilikatzeolithe des Pentasiltyps für das erfindungsgemäße Verfahren. Der Aluminosilikatzeolith wird z.B. aus einer Aluminiumverbindung, vorzugsweise $Al(OH)_3$ oder $Al_2(So_4)_3$ und einer Siliciumkomponente, vorzugsweise hochdispersem Siliciumdioxid in wäßriger Aminlösung, insbesondere in Polyaminen wie 1,6-Hexandiamin- oder 1,3-Propandiamin- oder Triethylentetramin-Lösung mit oder insbesondere ohne Alkali- oder Erdalkalizusatz bei 100 bis 220 °C unter autogenem Druck hergestellt. Auch gehören hierzu die isotaktischen Zeolithe nach EP O 034 727. Die erhaltenen Aluminosilikatzeolithe enthalten je nach Wahl der Einsatzstoffmengen ein $SiO_2/Al_2O_3$-Verhältnis von 10 bis 40 000. Auch lassen sich derartige Aluminosilikatzeolithe in etherischem Medium wie Diethylenglykoldimethylether, in alkoholischem Medium wie Methanol bzw. 1,4-Butandiol oder in Wasser synthetisieren.

Der Borosilikatzeolith wird z.B. bei 90 bis 200 °C unter autogenem Druck synthetisiert, indem man eine Borverbindung, z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Auch isotaktische Borosilikatzeolithe, hergestellt nach EP O 034 727 kann man vorteilhaft verwenden. Man kann auch Borosilikatzeolithe verwenden, die statt aus wäßriger Aminlösung aus einer etherischen Lösung, z.B. Diethylenglykoldimethylether oder aus alkoholischer Lösung, z.B. 1,6-Hexandiol auskristallisiert werden.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 200 °C unter autogenem Druck.

Zu den verwendbaren siliciumreichen Zeolithen ($SiO_2/Al_2O_3$ größer oder gleich 10) gehören auch die bekannten ZSM-Typen sowie Ferrierit und NU-1 sowie Silicalite®, ein Molekularsieb, ein sog. Silica Polymorph.

Die so hergestellten Alumino-, Boro- und Eisensilikatzeolithe können nach ihrer Isolierung, Trocknung bei 100 bis 160 °C, vorzugsweise 110 °C und Calcinierung bei 450 bis 550 °C, vorzugsweise 500 °C, mit einem Bindemittel im Verhältnis 90:10 bis 40:60 Gew.-% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25:75 bis 90:5, bevorzugt 75:25, Siliciumdioxid, bevorzugt hochdisperses $SiO_2$, $TiO_2$, Gemische aus hochdispersem $SiO_2$ und hochdispersem $Al_2O_3$ sowie Ton. Nach der Verformung werden die Extrudate oder Preßlinge bei 110 °C/16 h getrocknet und bei 500 °C/16 h calciniert.

Man erhält auch gut geeignete Katalysatoren, wenn der isolierte Alumino-bzw. Borosilikatzeolith direkt nach der Trocknung verformt wird und erst nach der Verformung einer Calcinierung unterworfen wird. Die Alumino- und Borosilikatzeolithe können in reiner Form, ohne Binder, als Stränge oder Tabletten eingesetzt werden, wobei als Verstrangungs oder Peptisierungsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden kann.

Liegt der Zeolith aufgrund der Art seiner Herstellung nicht in der katalytisch aktiven, aciden H-Form vor, sondern, z.B. in der Na-Form, dann kann diese durch Ionenaustausch, z.B. mit Ammoniumionen, und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eine durch Koksabscheidung bedingte Desaktivierung eintritt, empfiehlt es sich, die Zeolithe durch Abtrennen der Koksablagerung mit Luft oder mit einem Luft/$N_2$-Gemisch bei 400 bis 550 °C, bevorzugt 500 °C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, ist es vorteilhaft, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden Alkalimetall wie Li, Cs, Erdalkalimetalle wie Mg, Ca, Übergangsmetalle wie Cu, Zn, Cr, Mn, Fe, Co, Ni, W und Mo und Edelmetalle wie Pd, Pt, Seltene Erdmetalle wie Ce, La eingesetzt.

Zweckmäßigerweise führt man die Dotierung so durch, daß man z.B. den verformten Zeolithen in einem Steigrohr vorlegt und bei 20 bis 100 °C, z.B. eine wäßrige oder ammoniakalische Lösung eines Halogenids oder eines Nitrats der voranbeschriebenen Metalle überleitet. Ein derartiger Ionenaustausch kann z.B. an der Wasserstoff-, Ammonium-, Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man $Cu(NO_3)_2 \times 3\ H_2O$ oder $Co(NO_3)_2 \times 6\ H_2O$ oder $Ce(NO_3)_3 \times 6\ H_2O$ oder $La(NO_3)_3 \times 6\ H_2O$ oder $Cs_2CO_3$ in Wasser löst. Mit dieser Lösung wird der verformte oder unverformte Zeolith eine gewisse Zeit, ca. 30 Minuten, getränkt. Die eventuelle überstehende Lösung wird am Rotationsverdampfer von Wasser befreit. Danach wird der getränkt Zeolith bei ca. 150 °C getrocknet und bei 550 °C calciniert. Dieser Tränkvorgang kann mehrmals hintereinander vorgenommen werden, um den gewünschten Metallgehalt einzustellen.

Auch ist es möglich, z.B. eine wäßrige $Co(NO_3)_2$-Lösung oder ammoniakalische $Pd(NO_3)_2$-Lösung herzustellen und darin den reinen pulverförmigen Zeolithen bei 40 bis 100 °C unter Rühren ca. 24 h aufzuschlämmen. Nach Abfiltrieren, Trocknen bei ca. 150 °C und Calcinierung bei ca. 500 °C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man der Zeolithen in Strängen oder Pellets in einer Kolonne vorlegt und darüber z.B. eine wäßrige $Co(NO_3)_2$-Lösung oder ammoniakalische $Pd(NO_3)_2$-Lösung bei leicht erhöhter Temperatur zwischen 30 und 80 °C im Kreislauf 15 bis 20 h leitet. Danach wird mit Wasser ausgewaschen, bei ca. 150 °C getrocknet und bei ca. 550 °C calciniert.

Bei manchen metalldotierten Zeolithen z.B. Pd-, Cu-, Ni-dotierten Zeolithen ist eine Nachbehandlung

mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft z.B. so vor, daß man Zeolithe in Pulverform mit 1 n Phosphorsäure 1 h bei 80 °C behandelt. Nach der Behandlung wird mit Wasser gewaschen, bei 110 °C/16 h getrocknet und bei 500 °C/20 h calciniert. Nach einer anderen Arbeitsweise behandelt man Zeolithe vor oder nach ihrer Verformung mit Bindemitteln, z.B. 1 bis 3 h bei Temperaturen von 60 bis 80 °C mit einer 3- bis 25 gew.%igen, insbesondere 12- bis 20 gew.%igen wäßrigen Salzsäure. Anschließend wird der so behandelte Zeolith mit Wasser gewaschen, getrocknet und bei 400 bis 500 °C calciniert.

Eine besondere Ausführungsform für die Säurebehandlung besteht darin, daß man das zeolithische Material vor seiner Verformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von im allgemeinen 0,5 bis 5, vorzugsweise 1 bis 3 Stunden. Nach Isolierung, z.B. durch Abfiltrieren und Auswaschen, des zeolithischen Materials wird dieses zweckmäßig, z.B. bei Temperaturen von 100 °C bis 160 °C, getrocknet und bei Temperaturen von im allgemeinen 450 °C bis 600 °C calciniert. Gemäß einer weiteren bevorzugten Ausführungsform für die Säurebehandlung wird das zeolithische Material nach seiner Verformung mit Bindemittel bei erhöhter Temperatur, zweckmäßig bei Temperaturen von 50 °C bis 90 °C, vorzugsweise 1 bis 3 h mit Salzsäure, im allgemeinen mit 3- bis 25 gew.%iger Salzsäure, vorzugsweise mit 12- bis 20 gew.%iger Salzsäure, behandelt. Anschließend wird das zeolithische Material im allgemeinen ausgewaschen und zweckmäßig, z.B. bei Temperaturen von 100 bis 160 °C, getrocknet und bei Temperaturen von im allgemeinen 450 °C bis 600 °C calciniert. Auch eine aufeinanderfolgende Behandlung mit HF und HCl ist gegebenenfalls vorteilhaft.

Nach einer anderen Arbeitsweise lassen sich Zeolithe durch Aufbringung von Phosphorverbindungen, wie Trimethylphosphat, Trimethoxyphosphin, primärem, sekundärem oder tertiärem Natriumphosphat modifizieren. Besonders vorteilhaft hat sich die Behandlung mit primärem Natriumphosphat erwiesen. Hierbei werden die Zeolithe in Strang-, Tabletten- oder Wirbelgut-Form mit wäßriger $NaH_2PO_4$-Lösung getränkt, bei 110 °C getrocknet und bei 500 °C calciniert.

Die hier beschriebenen Katalysatoren können wahlweise als 2- bis 4 mm-Stränge oder als Tabletten mit 3 bis 5 mm Durchmesser oder als Splitt mit Teilchengrößen von 0,1 bis 0,5 mm oder als Wirbelkontakt eingesetzt werden.

Die für die erfindungsgemäße Umwandlung in der Regel gewählten Reaktionsbedingungen liegen bei der bevorzugten Gasphasenreaktion bei 100 bis 500 °C, vorzugsweise 150 bis 300 °C, und einer Belastung WHSV von 0,1 bis 20 $H^{-1}$, bevorzugt 0,5 bis 5 $h^{-1}$ (g Einsatzgemisch/g Katalysator und Stunde).

Es ist aber auch möglich, die Reaktion in der Flüssigphase in Suspensions-, Riesel- oder Sumpffahrweise bei Temperaturen zwischen 50 und 300 °C, bevorzugt zwischen 100 und 250 °C und einer Belastung g Edukt : g Katalysator = 100:1 bis 5:1, bevorzugt 60:1 bis 10:1 durchzuführen.

Das Verfahren wird in der Regel bei Normaldruck oder je nach Flüchtigkeit der Ausgangsverbindung bei vermindertem Druck oder erhöhtem Druck durchgeführt. Die Durchführung erfolgt vorzugsweise kontinuierlich.

Schwerflüchtige oder feste Edukte werden in gelöster Form, z.B. in THF-, Toluol- oder Petrolether-Lösung, zum Einsatz gebracht. Generell ist eine Verdünnung des Eduktes mit derartigen Lösungsmitteln oder mit Inertgasen wie $N_2$, Ar, He oder mit $H_2O$-Dampf möglich.

Nach der Umsetzung werden die entstandenen acylierten Heteroaromaten durch übliche Techniken, z.B. durch Destillation aus dem Reaktionsgemisch isoliert; nichtumgesetztes Einsatzgemisch wird gegebenenfalls - wenn nötig - für die erfindungsgemäße Umsetzung zurückgeführt.

Die folgenden Beispiele veranschaulichen die Erfindung.

Beispiele 1 - 23

Die Reaktionen in der Gasphase wurden unter isothermen Bedingungen in einem Rohrreaktor (Wendel, 0,6 cm Innendurchmesser, 90 cm Länge) mindestens 6 h lang durchgeführt. Die Reaktionsprodukte wurden durch übliche Methoden abgetrennt und charakterisiert. Die quantitative Bestimmung der Reaktionsprodukte und der Ausgangsprodukte erfolgte gaschromatographisch.

Die für das erfindungsgemäße Verfahren eingesetzten Katalysatoren sind:

Katalysator A

Der Borosilikatzeolith des Pentasil-Typs wird in einer hydrothermalen Synthese aus 640 g hochdisper-

sem $SiO_2$, 122 g $H_3BO_3$, 8000 g einer wäßrigen 1,6-Hexandiamin-Lösung (Mischung 50:50 Gew.%) bei 170 °C unter autogenem Druck in einem Rührautoklaven hergestellt. Nach Abfiltrieren und Auswaschen wird das kristalline Reaktionsprodukt bei 100 °C/24 h getrocknet und bei 500 °C/24 h calciniert. Dieser Borosilikatzeolith setzt sich zusammen aus 94,2 Gew.-% $SiO_2$ und 2,3 Gew.-% $B_2O_3$.

Mit diesem Material werden durch Verformen mit einem Verformungshilfsmittel 2-mm-Stränge hergestellt, die bei 100 °C/16 h getrocknet und bei 500 °C/24 h calciniert werden.

Katalysator B

Katalysator B wird erhalten durch Verformung des Borosilikatzeolithen (Vgl. Katalysator A) mit Boehmit (Gewichtsverhältnis 60:40) zu 2-mm-Strängen, die bei 110 °C/16 h getrocknet und bei 500 °C/16 h calciniert werden.

Diese Stränge werden mit einer wäßrigen $Ce(NO_3)_3$-Lösung dotiert, bei 130 °C/2 h getrocknet und bei 500 °C/2 h calciniert. Dieser Katalysator B enthält 2,5 Gew.-% Ce.

Katalysator C

Katalysator C wird hergestellt aus Katalysator A durch Imprägnierung mit einer wäßrigen $Cs_2CO_3$-Lösung, anschließende Trocknung bei 110 °C/16 h und Calcination bei 500 °C/16 h. Der Cs-Gehalt beträgt 0,21 Gew.-%.

Katalysator D

Katalysator D wird hergestellt wie Katalysator C, nur wird $La(NO_3)_3$ anstelle von $Cs_2CO_3$ verwendet. Der La-Gehalt ist 1,6 Gew.-%.

Katalysator E

Katalysator E wird hergestellt wie Katalysator C, nur wird $Ce(NO_3)_3$ anstelle von $Cs_2CO_3$ verwendet. Der Ce-Gehalt ist 1,75 Gew.-%.

Katalysator F

Katalysator F wird präpariert, indem man den Katalysator A mit Trimethylphosphat gelöst in Toluol 16 h unter Rückfluß erhitzt. Der P-Gehalt beträgt nach Trocknung bei 110 °C/16 h und Calcination bei 500 °C/16 h 2,07 Gew.-%.

Katalysator G

Katalysator G wird wie Katalysator B hergestellt, wobei jedoch $Ce(NO_3)_3$-Lösung zusammen mit einer $Pd(NO_3)_2$-Lösung für die Dotierung verwendet wird. Der Ce-Gehalt beträgt 3,6 Gew.-%, der Pd-Gehalt 3,9 Gew.-%.

Katalysator H

Der bei Katalysator A hergestellte Borosilikatzeolith wird mit pyrogener Kieselsäure im Massenverhältnis 60:40 verstrangt, bei 110 °C/16 h getrocknet und bei 500 °C/16 h calciniert. Die Stränge werden mit $La(NO_3)_3$-Lösung wie bei Katalysator D getränkt (La = 3,25 Gew.-%).

Katalysator I

Ein Aluminosilikatzeolith vom Pentasil-Typ wurde unter hydrothermalen Bedingungen bei autogenem Druck und 150 °C aus 650 g hochdispersem $SiO_2$, 203 g $Al_2(So_4)_3 \times 18\ H_2O$ in 10 kg einer wäßrigen 1,6-Hexandiamin-Lösung (Mischung 50:50 Gew.-% in einem Rührautoklaven hergestellt. Nach Abfiltrieren und Auswaschen wurde das kristalline Reaktionsprodukt bei 110 °C/24 h getrocknet und bei 500 °C/24 h calciniert. Dieser Aluminosilikatzeolith enthielt 92,8 Gew.-% $SiO_2$ und 4,2 Gew.-% $Al_2O_3$. Mit diesem Material werden durch Verformen mit einem Verformungshilfsmittel 2-mm-Stränge hergestellt, die bei 110 °C/16 h getrocknet und bei 500 °C/24 h calciniert werden.

Katalysator J

Katalysator J wird hergestellt, indem man eine wäßrige $Ce(NO_3)_3$-Lösung auf Katalysator I aufbringt, bei 130°C/2 h trocknet und bei 540°C/2 h calciniert. Katalysator J besitzt 6,5 Gew.-% Ce.

Katalysator K

Katalysator K wird erhalten, indem man Katalysator I mit 48 %iger Phosphorsäure tränkt. Der P-Gehalt beträgt 7,96 Gew.-%.

Katalysator L

Zur Herstellung des Katalysators L wurde der gemäß I hergestellte Alusilikatzeolith mit HF behandelt. Hierbei wurden 50 g des Alusilikatzeolithen mit 140 ml 0,1 n HF unter Rückfluß gekocht. Nach Abfiltrieren wurde mit Wasser neutral gewaschen, bei 110°C/16 h getrocknet und bei 500°C/5 h calciniert. Dieses Material wurde mit amorphem Aluminosilikat ($SiO_2$:$Al_2O_3$ = 75:25 Gew.-%) im Massenverhältnis 60:40 verstrangt. Danach wurde bei 100°C/16 h getrocknet und bei 500°C/16 h calciniert.

Die mit diesen Katalysatoren erhaltenen Ergebnisse und die Versuchsbedingungen sind in Tabelle 1-3 aufgeführt.

Tabelle 1: Acetylierung von Furan (Furan:Essigsäureanhydrid = 2:1 molar)

| Beispiel | Katalysator | Temp. [°C] | WHSV [$h^{-1}$] | Umsatz Furan [%] | Selektivität 2-Acetyl-furan [%] |
|---|---|---|---|---|---|
| 1 | A | 200 | 3,7 | 11 | 99 |
| 2 | E | 200 | 2,2 | 23 | 99 |
| 3 | D | 200 | 4,0 | 17 | 99 |
| 4 | H | 200 | 3,9 | 19 | 99 |
| 5 | B | 200 | 3,8 | 19 | 99 |
| 6 | B | 150 | 3,9 | 8 | 99 |
| 7 | G | 200 | 3,9 | 10 | 99 |
| 8 | L | 200 | 3,8 | 9 | 99 |
| 9 | I | 150 | 3,0 | 12 | 99 |

Tabelle 2: Acetylierung von Thiophen (Thiophen:Essigsäureanhydrid = 3:1 molar)

| Beispiel | Katalysator | Temp. [°C] | WHSV [$h^{-1}$] | Umsatz Thiophen [%] | Selektivität 2-Acetyl-thiophen [%] |
|---|---|---|---|---|---|
| 10 | A | 200 | 3,1 | 13 | 99 |
| 11 | A | 250 | 3,1 | 24 | 99 |
| 12 | D | 200 | 3,1 | 9 | 99 |
| 13 | I | 150 | 3,2 | 7 | 99 |

Tabelle 3: Acetylierung von Pyrrol (Pyrrol:Essigsäureanhydrid = 2:1 molar)

| Beispiel | Katalysator | Temp. [°C] | WHSV [$h^{-1}$] | Umsatz Pyrrol [%] | Selektivität 2-Acetyl-pyrrol [%] |
|---|---|---|---|---|---|
| 14 | C | 150 | 3,4 | 41 | 98 |
| 15 | A | 150 | 2,8 | 40 | 98 |
| 16 | L | 150 | 3,2 | 17 | 92 |
| 17 | K | 150 | 3,0 | 14 | 91 |
| 18 | I | 150 | 3,2 | 10 | 89 |

Tabelle 4: Acylierung von Furan mit Propionsäure (Furan:Propionsäure = 1:1 molar)

| Beispiel | Katalysator | Temp. [°C] | WHSV [$h^{-1}$] | Umsatz Furan [%] | Selektivität 2-Furylethylketon [%] |
|---|---|---|---|---|---|
| 19 | I | 250 | 4,1 | 35 | 98 |
| 20 | A | 250 | 4,7 | 30 | 98 |
| 21 | E | 250 | 6,2 | 29 | 98 |

Beispiele 22-23

In einem Autokolaven werden 118 g eines Gemisches von Thiophen und Essigsäureanhydrid (3:1, molar) unter Zugabe von 2 g Katalysator I unter Rühren 8 h bei Eigendruck auf 200 °C erhitzt. Die gaschromatographsiche Analyse der Produktgemisches ergibt einen Umsatz des Thiophens von 32 % und

eine Selektivität bezüglich 2-Acetylthiophen von 98 %.

Bei Verwendung des Katalysators F unter sonst identischen Bedingungen wird ein Umsatz von 27 % und eine Selektivität von 98 % gemessen.

## Patentansprüche

1. Verfahren zur Herstellung von acylierten Heteroaromaten der Formel Ia oder Ib

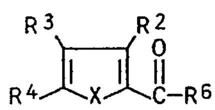

(Ia),

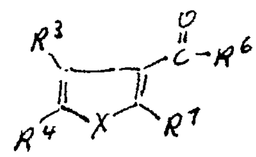

(Ib)

worin
X = O oder $NR^5$,
$R^1$-$R^4$ = H-, Alkyl- mit 1 bis 12 Kohlenstoffatomen, Alkenyl- mit bis 12 Kohlenstoffatomen, Alkoxy- mit 1 bis 12 Kohlenstoffatomen, Aryl-, Aralkyl- oder Alkylarylreste,
$R^5$ = H-, Alkyl- mit 1 bis 12 Kohlenstoffatomen, Alkenyl- mit bis 12 Kohlenstoffatomen, Aryl-, Aralkyl- oder Alkylarylreste und
$R^6$ = H-, Alkyl- mit 1 bis 12 Kohlenstoffatomen, Alkenyl- mit bis 12 Kohlenstoffatomen, Aryl-, Aralkyl-, Alkylaryl- oder Carboxyreste bedeuten,
dadurch gekennzeichnet, daß man fünfgliedrige Heteroaromaten der Formel II

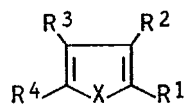

(II),

in der
X = O oder $NR^5$,
$R^1$-$R^4$ = H-, Alkyl- mit 1 bis 12 Kohlenstoffatomen, Alkenyl- mit bis 12 Kohlenstoffatomen, Alkoxy- mit 1 bis 12 Kohlenstoffatomen, Aryl-, Aralkyl- oder Alkylarylreste bedeuten und
$R^5$ die obengenannte Bedeutung hat,
wobei mindestens einer der Reste $R^1$ und $R^2$ Wasserstoff ist,
mit Acylierungsmitteln der Formel III

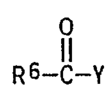

(III),

in der
$R^6$ die obengenannte Bedeutung hat und
Y einen Halogenid-, Alkoxy-, Acyloxy-, Hydroxy-, Amid- oder Carboxyreste bedeuten,
in Gegenwart von Zeolithen als Katalysatoren umsetzt.

2. Verfahren nach Anspruch 1, dadurch gekennzeichnet, daß man als Katalysatoren Zeolithe des Pentasil-Typs verwendet.

3. Verfahren nach Anspruch 1, dadurch gekennzeichnet, daß man als Katalysatoren Aluminosilikatzeolithe verwendet.

4. Verfahren nach Anspruch 1, dadurch gekennzeichnet, daß man als Katalysatoren Borosilikatzeolithe verwendet.

5. Verfahren nach Anspruch 1, dadurch gekennzeichnet, daß man als Katalysatoren Eisensilikatzeolithe verwendet.

6. Verfahren nach Anspruch 1, dadurch gekennzeichnet, daß man als Katalysatoren Zeolithe des Faujasit-Typs verwendet.

7. Verfahren nach Anspruch 1, dadurch gekennzeichnet, daß man als Katalysatoren ultrastabile Zeolithe des Faujasit-Typs verwendet.

8. Verfahren nach Anspruch 1, dadurch gekennzeichnet, daß man als Katalysatoren mit Alkali- und/oder Erdalkalimetall dotierte Zeolithe verwendet.

9. Verfahren nach Anspruch 1, dadurch gekennzeichnet, daß man mit Übergangsmetallen dotiert Zeolithe als Katalysatoren verwendet.

10. Verfahren nach Anspruch 1, dadurch gekennzeichnet, daß man mit seltenen Erdmetallen dotierte Zeolithe als Katalysatoren verwendet.

11. Verfahren nach Anspruch 8, dadurch gekennzeichnet, daß man als Katalysatoren mit Caesium dotiert Zeolithe verwendet.

12. Verfahren nach Anspruch 10, dadurch gekennzeichnet, daß man als Katalysatoren Ce und/oder La dotierte Zeolithe verwendet.

13. Verfahren nach Anspruch 1, dadurch gekennzeichnet, daß man als Katalysatoren mit Säuren behandelte Zeolithe verwendet.

14. Verfahren nach Anspruch 1, dadurch gekennzeichnet, daß man als Katalysatoren mit Phosphor dotierte Zeolithe verwendet.

## Claims

1. A process for the preparation of an acylated heteroaromatic of the formula Ia or Ib

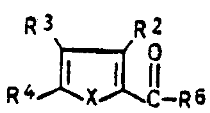 (Ia), 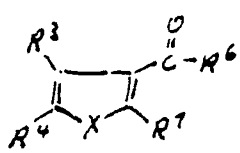 (Ib)

where
X is O or $NR^5$,
$R^1$, $R^2$, $R^3$ and $R^4$ are each H, alkyl or alkoxy each of 1 to 12 carbon atoms, alkenyl of up to 12 carbon atoms, aryl, aralkyl or alkylaryl,
$R^5$ is H, alkyl of 1 to 12 carbon atoms, alkenyl of up to 12 carbon atoms, aryl, aralkyl or alkylaryl and
$R^6$ is H, alkyl of 1 to 12 carbon atoms, alkenyl of up to 12 carbon atoms, aryl, aralkyl, alkylaryl or carboxyl, wherein a five-membered heteroaromatic of the formula II

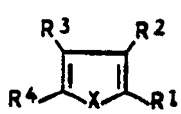 (II),

where
X is O or $NR^5$,
$R^1$, $R^2$, $R^3$ and $R^4$ are each H, alkyl or alkoxy, each of 1 to 12 carbon atoms, alkenyl of up to 12 carbon

atoms, aryl, aralkyl or alkylaryl and
$R^5$ has the above meanings, one or more of the radicals $R^1$ or $R^2$ being hydrogen, is reacted with an acylating agent of the formula III

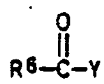 (III),

where
$R^6$ has the above meanings and Y is halide, alkoxy, acyloxy, hydroxyl, amide or carboxyl, in the presence of a zeolite as a catalyst.

**2.** A process as claimed in claim 1, wherein the catalyst used is a zeolite of pentasil type.

**3.** A process as claimed in claim 1, wherein the catalyst used is an aluminosilicate zeolite.

**4.** A process as claimed in claim 1, wherein the catalyst used is a borosilicate zeolite.

**5.** A process as claimed in claim 1, wherein the catalyst used is an iron silicate zeolite.

**6.** A process as claimed in claim 1, wherein the catalyst used is a zeolite of the faujasite type.

**7.** A process as claimed in claim 1, wherein the catalyst used is an ultrastable zeolite of the faujasite type.

**8.** A process as claimed in claim 1, wherein the catalyst used is a zeolite which is doped with an alkali metal and/or alkali earth metal.

**9.** A process as claimed in claim 1, wherein the catalyst used is a zeolite doped with transition metals.

**10.** A process as claimed in claim 1, wherein the catalyst used is a zeolite doped with rare earth metals.

**11.** A process as claimed in claim 8, wherein the catalyst used is a cesium-doped zeolite.

**12.** A process as claimed in claim 10, wherein the catalyst used is a zeolite doped with Ce and/or La.

**13.** A process as claimed in claim 1, wherein the catalyst used is a zeolite which has been treated with an acid.

**14.** A process as claimed in claim 1, wherein the catalyst used is a phosphorus-doped zeolite.

**Revendications**

**1.** Procédé de préparation de composés hétéroaromatiques acylés de la formule Ia ou de la formule Ib

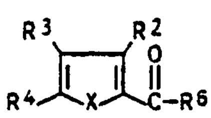 Ia, 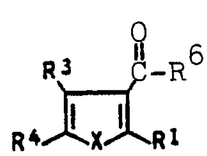 Ib

dans lesquelles
X = O ou $NR^5$,
$R^1$-$R^4$ = H, restes alkyle contenant 1 à 12 atomes de carbone, alcényle contenant jusqu'a 12 atomes de carbone, alcoxy contenant 1 à 12 atomes de carbone, aryle, aralkyle ou alkylaryle,

$R^5$ = H, restes alkyle contenant 1 à 12 atomes de carbone, alcényle contenant Jusqu'à 12 atomes de carbone, aryle, aralkyle, ou alkylaryle et
$R^6$ = H, restes alkyle contenant 1 à 12 atomes de carbone, alcényle contenant jusqu'à 12 atomes de carbone, aryle, aralkyle, alkylaryle ou carboxy,
caractérisé en ce que l'on fait réagir, en présence de zéolites comme catalyseurs, des composés hétéroaromatiques a cinq chaînons de la formule II

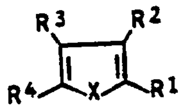

II,

dans laquelle
X = O ou $NR^5$,
$R^1$-$R^4$ = H, restes alkyle contenant 1 à 12 atomes de carbone, alcényle contenant jusqu'à 12 atomes de carbone, alcoxy contenant 1 a 12 atomes de carbone, aryle, aralkyle ou alkylaryle et
$R^5$ a les significations susindiquées,
l'un au moins des restes $R^1$ et $R^2$ étant de l'hydrogène, avec des agents d'acylation de la formule III

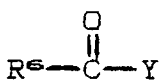

III,

dans laquelle $R^6$ a les significations susindiquées et Y désigne l'un des restes halogénure, alcoxy, acyloxy, hydroxy, amide ou carboxy.

2. Procédé suivant la revendication 1, caractérisé en ce que l'on utilise comme catalyseurs des zéolites du type pentasil.

3. Procédé suivant la revendication 1, caractérisé en ce que l'on utilise comme catalyseurs des zéolites qui sont des aluminosilicates.

4. Procédé suivant la revendication 1, caractérisé en ce que l'on utilise comme catalyseurs des zéolites qui sont des borosilicates.

5. Procédé suivant la revendication 1, caractérisé en ce que l'on utilise comme catalyseurs des zéolites qui sont des ferrosilicates.

6. Procédé suivant la revendication 1, caractérisé en ce que l'on utilise comme catalyseurs des zéolites du type faujasite.

7. Procédé suivant la revendication 1, caractérisé en ce que l'on utilise comme catalyseurs des zéolites ultrastables du type faujasite.

8. Procédé suivant la revendication 1, caractérisé en ce que l'on utilise comme catalyseurs des zéolites dopées avec des métaux alcalins et/ou alcalino-terreux.

9. Procédé suivant la revendication 1, caractérisé en ce que l'on utilise comme catalyseurs des zéolites dopées avec des métaux de transition.

10. Procédé suivant la revendication 1, caractérisé en ce que l'on utilise comme catalyseurs des zéolites dopées avec des métaux des terres rares.

11. Procédé suivant la revendication 8, caractérisé en ce que l'on utilise comme catalyseurs des zéolites dopées avec du césium.

12. Procédé suivant la revendication 10, caractérisé en ce que l'on utilise comme catalyseurs des zéolites dopées avec Ce et/ou La.

13. Procédé suivant la revendication 1, caractérisé en ce que l'on utilise comme catalyseurs des zéolites traitées par des acides.

14. Procédé suivant la revendication 1, caractérisé en ce que l'on utilise comme catalyseurs des zéolites dopées avec du phosphore.